# EUROPÄISCHE PATENTSCHRIFT

(11) **EP 2 087 915 B1**
(45) Veröffentlichungstag und Bekanntmachung des Hinweises auf die Patenterteilung: **01.07.2015**
(21) Anmeldenummer: 08172105.2
(22) Anmeldetag: 18.12.2008
(51) Int. Cl.: A61L 31/02, A61L 31/14

(54) **Implantat mit einem Grundkörper aus einer biokorrodierbaren Eisenlegierung**
Implant with a base body made of a biocorrodible iron alloy
Implant doté d'un corps de base constitué d'un alliage de fer biocorrodable

(30) Priorität: 05.02.2008 DE 102008007746; 24.06.2008 DE 102008002601
(43) Veröffentlichungstag der Anmeldung: 12.08.2009
(73) Patentinhaber: Biotronik VI Patent AG, 6341 Baar (CH)
(72) Erfinder: Müller, Heinz, 91052, Erlangen (DE); Löffler, Jörg, 5425, Schneisingen (CH); Uggowitzer, Peter, 8913, Ottenbach (CH)
(74) Vertreter: Lindner-Vogt, Karin L.

(56) Entgegenhaltungen:
- EP-A1- 0 540 483
- US-A- 5 197 940
- HENDRA HERMAWAN ET AL: "Degradation Behaviour of Metallic Biomaterials for Degradable Stents", ADVANCED MATERIALS RESEARCH,, Bd. 15-17, 1. Januar 2007 (2007-01-01), Seiten 113-118, XP008157554, ISSN: 1022-6680, DOI: 10.4028/WWW.SCIENTIFIC.NET/AMR.15-17.113 [gefunden am 2006-02-15]

## Beschreibung

Die Erfindung betrifft ein Implantat mit einem Grundkörper, der ganz oder in Teilen aus einer biokorrodierbaren Eisenlegierung besteht.

### Technologischer Hintergrund und Stand der Technik

Implantate finden in vielfältiger Ausführungsform in der modernen Medizintechnik Anwendung. Sie dienen unter anderem der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

Die Implantation von Stents ist eine der wirkungsvollsten therapeutischen Maßnahmen bei der Behandlung von Gefäßerkrankungen. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper jedes Implantats, insbesondere von Stents, besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Für die Zwecke der vorliegenden Erfindung sind lediglich degradierbare/resorbierbare, metallische Implantatwerkstoffe von Interesse, die nachfolgend als biokorrodierbare metallische Werkstoffe bezeichnet werden.

Der Einsatz biokorrodierbarer metallischer Werkstoffe bietet sich insbesondere schon deshalb an, da häufig nur ein zeitweiliger Verbleib des Implantats im Körper zur Erfüllung des medizinischen Zweckes erforderlich ist. Implantate aus permanenten Werkstoffen, also Werkstoffen, die im Körper nicht abgebaut werden, sind gegebenenfalls wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht demnach darin, das Implantat ganz oder in Teilen aus einem biokorrodierbaren metallischen Werkstoff zu formen. Unter Biokorrosion werden Vorgänge verstanden, die durch die Anwesenheit von Körpermedien bedingt sind und die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Die Abbauprodukte haben, wie beispielsweise beim Magnesium, im günstigsten Fall sogar eine positive therapeutische Wirkung auf das umgebende Gewebe. Geringe Mengen nicht resorbierbarer Legierungsbestandteile sind - sofern sie nicht toxisch sind - tolerierbar.

Bekannte biokorrodierbare metallische Werkstoffe umfassen Reineisen und biokorrodierbare Legierungen der Hauptelemente Magnesium, Eisen, Zink, Molybdän und Wolfram. In DE 197 31 021 A1 wird unter anderem vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Insbesondere limitiert die relativ langsame Biokorrosion von Reineisen oder der bekannten Eisenlegierungen deren Einsatzmöglichkeiten.

Herkömmliche technische Einsatzgebiete von Formkörpern aus metallischen Werkstoffen, insbesondere aus Eisenlegierungen außerhalb der Medizintechnik, erfordern in der Regel eine weitgehende Unterbindung korrosiver Prozesse. Dementsprechend ist die Zielstellung der meisten technischen Verfahren zur Verbesserung des Korrosionsverhaltens eine vollständige Inhibierung korrosiver Prozesse. Dagegen sollte die Zielstellung zur Verbesserung des Korrosionsverhaltens der hier vorliegenden biokorrodierbaren Eisenlegierungen nicht in der vollständigen Unterbindung, sondern gerade in einer Beschleunigung der korrosiven Prozesse liegen. Ferner müssen für einen medizintechnischen Einsatz auch toxikologische Aspekte berücksichtigt werden. Schließlich sind korrosive Prozesse stark von dem Medium abhängig, in dem sie ablaufen; eine Übertragung der unter herkömmlichen Umweltbedingungen auf technischem Gebiet gewonnenen Erkenntnisse über die Eigenschaften spezifischer Eisenlegierungen auf die Prozesse in physiologischer Umgebung ist daher in der Regel nicht möglich.

WO 2007/082147 A2 befasst sich mit bioerodierbaren Endoprothesen, die mindestens zwei Abschnitte aus metallischen Werkstoffen mit unterschiedlicher Korrosionsrate aufweisen. Beispielhaft werden Eisenlegierungen der Zusammensetzung (i) 88 bis 99,8 Gew.% Fe, 0,1 - 7 Gew.% Cr, 0 - 3,5 Gew.% Ni und weniger als 5 Gew.% anderer Elemente sowie (ii) 90 bis 96 Gew.% Fe, 3 - 6 Gew.% Cr, 0 - 3 Gew.% Ni und 0 - 5 Gew.% andere Metalle genannt. Bei Verwendung der Elemente Cr und Ni bei einem bioresorbierbaren Werkstoff ist mit einer erheblichen Beeinträchtigung der Biokompatibilität zu rechnen; speziell Chrom gehört zu den Elementen mit sehr großem Toxizitätspotenzial.

Hendra Hermawan et al. beschreiben das Degradationsverhalten eines Stents aus der Legierung Fe-35Mn (Advanced Materials Research, Vols. 15 - 17, (2007), pp. 113 - 116). Die Legierung zeigt jedoch nur eine allenfalls marginal erhöhte Korrosion. Nachteilig ist ferner, dass der relativ hohe Mn-Anteil in einem einphasigen Gefüge eine verringerte Duktilität erwarten lässt.

Ein weiterer Nachteil einer Legierung mit 35 Gew.% Mn ist, dass beim Abkühlen keine Umwandlung in den so genannten ε (Epsilon)-Martensit mehr möglich ist.

Ein weiterer Nachteil einer Legierung mit 35% Mn ist, dass sich hier kein zweiphasiges Gefüge einstellen lässt. Ein feinkörniges zweiphasiges Gefüge ist aber Basis einer außerordentlich guten Verformbarkeit. Ein Nachteil des Verlustes dieser Mehrphasigkeit ist, dass die Austenitstabilität nicht mehr mit Kohlenstoff oder Stickstoff eingestellt werden kann.

Biokorrodierbare Legierungen aus Eisen und Kohlenstoff sind ebenfalls bekannt (EP 0 923 389 B1 und WO 2007/124230 A1). Ein Nachteil dieser Legierungen ist, dass das reine Zweistoffsystem aus Eisen und Kohlenstoff mit zunehmendem Kohlenstoffgehalt sehr stark an Duktilität verliert, ohne dass in gleichem Maß die Korrosionsbeständigkeit absinkt.

Der vorliegenden Erfindung liegt die Aufgabe zugrunde, eine hinsichtlich ihres Korrosionsverhaltens verbesserte biokorrodierbare Eisenlegierung für ein Implantat bereitzustellen. Dies soll insbesondere derart erfolgen, dass die weiteren, für die Bearbeitung wichtigen Materialeigenschaften, wie zum Beispiel seine Duktilität, nicht verschlechtert werden.

### Zusammenfassung der Erfindung

Mit Hilfe des erfindungsgemäßen Implantats lassen sich ein oder mehrere der zuvor geschilderten Nachteile des Standes der Technik überwinden oder zumindest mindern. Ein Grundkörper des Implantats besteht dabei ganz oder in Teilen
(i) aus einer biokorrodierbaren Eisenlegierung der Formel (1):

   Fe-P (1)

   wobei ein Anteil von P an der Legierung 0,01 bis 5 Gew.% beträgt und Fe sowie herstellungsbedingte Verunreinigungen den auf 100 Gew.% verbleibenden Restanteil an der Legierung einnehmen; oder
(ii) aus einer biokorrodierbaren Eisenlegierung der Formel (2):

   Fe-Mn-X (2)

   wobei ein Anteil von Mn an der Legierung 5 bis 30 Gew.% beträgt, X für ein oder mehrere Elemente ausgewählt aus der Gruppe Pt, Pd, Ir, Rh, Re, Ru und Os steht und ein Anteil von X an der Legierung 0,01 bis 10 Gew.% beträgt und Fe sowie herstellungsbedingte Verunreinigungen den auf 100 Gew.% verbleibenden Restanteil an der Legierung einnehmen; oder
(iii) aus einer biokorrodierbaren Eisenlegierung der Formel (3):

   Fe-Z (3)
wobei Z für ein oder mehrere Elemente ausgewählt aus der Gruppe Pt, Ir und Os steht und ein Anteil von Z an der Legierung 5 bis 30 Gew.% beträgt und Fe sowie herstellungsbedingte Verunreinigungen den auf 100 Gew.% verbleibenden Restanteil an der Legierung einnehmen.

Es hat sich gezeigt, das Eisenlegierungen der Formeln (1) bis (3) eine gegenüber Reineisen deutlich erhöhte Korrosionsanfälligkeit in einem den physiologischen Bedingungen nachempfundenen künstlichen Medium zeigen.

Weiterhin hat sich überraschenderweise herausgestellt, dass in den Gefügen der Eisenlegierungen der Formeln (2) und (3) im vermehrten Maße zweiphasige Bereiche vorliegen. Diese sind zum Teil paramagnetisch, so dass sich die magnetischen Eigenschaften des metallischen Werkstoffs gegenüber zum Beispiel Reineisen erheblich ändern. Damit einhergehend werden Wechselwirkungen des metallischen Werkstoffs mit magnetischen Feldern verringert, was insbesondere eine Artefaktbildung im Kernspintomographen verkleinert.

Die Eisenlegierungen sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Beim System Fe-P wird durch das Zulegieren von Phosphor die Korrosionsbeständigkeit kontinuierlich abgesenkt. In Versuchen konnte die Korrosionsbeständigkeit durch Zulegieren von bis zu 3 At.% Phosphor im künstlichen Plasma (SBF; simulated body fluid) um bis zu Faktor 3 gegenüber Reineisen abgesenkt werden. Mit zunehmendem P-Gehalt nimmt die Festigkeit zu und die Verformbarkeit ab.

| SBF | [mmol/l] |
|---|---|
| Na⁺ | 142.0 |
| K⁺ | 5.0 |
| Mg²⁺ | 1.0 |
| Ca²⁺ | 2.5 |
| Cl⁻ | 109.0 |
| HCO₃⁻ | 27.0 |
| HPO₄²⁻ | 1.0 |
| SO₄²⁻ | 1.0 |
| pH | 7.3-7.4 |

Wird Mangan (Mn) entsprechend Formel (2) reinem Eisen zulegiert, so kann ebenfalls die Korrosionsbeständigkeit verringert werden. In Versuchen wurde insbesondere bei einer Zulegierung von 10 bzw. 20 Gew.% Mn eine erhebliche Verringerung der Korrosionsbeständigkeit gemessen. Der Massenverlust einer in SBF ausgelagerten Probe betrug das 3,5 bis 6-fache gegenüber Reineisen. Vorzugsweise beträgt der Anteil an Mn in der Eisenlegierung der Formel (2) daher 10 bis 27 Gew.%.

Eine Besonderheit zeigt dabei die Gefügeausbildung in diesen Legierungssystemen. Unlegiertes Eisen besitzt ein kubisch-raumzentriertes (krz) alpha Gitter (auch α-Eisen oder Ferrit genannt), welches ferromagnetisch ist und deshalb u. a. auch für die starken Artefakte bei Bildgebung mit MRI-Verfahren verantwortlich ist. Mit zunehmendem Gehalt an Mangan wird die Ausbildung dieser krz-Phase stärker unterdrückt. Dies geschieht dadurch, dass bei ausreichend hohen Legierungsgehalten an Mangan zwei Phasen im thermodynamischen Gleichgewicht existieren können; die beschriebene krz-Phase und eine kubisch-flächenzentrierte (kfz) Phase, die sogenannte γ-Phase (auch Austenit genannt). Diese kfz-Phase ist paramagnetisch und verursacht damit die o. g. Artefakte nicht oder nur in ganz geringem Umfang. Die Gefügeausbildung erfolgt dergestalt, dass nicht mehr alle Gefügebestandteile (Körner) die krz-Phase ausbilden, sondern mit zunehmendem Mn-Gehalt immer mehr Körner eine kubisch-flächenzentrierte Struktur besitzen. Ein solches Gefüge mit zwei im Gleichgewicht existierenden Phasen nennt man Duplexgefüge.

Ab Mn-Gehalten im Bereich von 30 Gew.% wird das Gefüge vollständig austenitisch. Dabei ist zu beachten, dass je nach Abkühlgeschwindigkeit bei der Herstellung der Legierung die Umwandlung der kfz-Phase in die krz-Phase bzw. in ein entsprechendes Duplexgefüge auch bei Mn-Gehalten deutlich unter 30% unterdrückt werden kann und dann ein rein austenitisches Gefüge vorliegt. Die Ausbildung des o. g. Duplexgefüges hat verschiedene Auswirkungen. Einmal wird mit zunehmendem kfz-Anteil die Wechselwirkung mit magnetischen Feldern geringer und speziell auch die verursachten Artefakte in Kernspintomographen kleiner. Ein weiterer Effekt der beiden nebeneinander vorliegenden Phasen ist, dass die Existenz einer elektrochemisch edleren und einer unedleren Phase eine korrosionsbeschleunigende Wirkung hat. Darüber hinaus können diese zweiphasigen Gefüge sehr feinkörnig hergestellt werden und sind auch sehr stabil gegen Kornwachstum; d. h. sie bleiben auch bei thermomechanischen Behandlungen feinkörnig und damit auch duktil. So ist auch trotz einer mit zunehmendem Mn-Gehalt ansteigenden Härte eine gute Umformbarkeit und eine gute Duktilität zu erwarten.

Dadurch, dass sich bei diesen Werkstoffen, bei geeignet eingestellten Legierungsgehalten zwei Phasen ausbilden, von denen eine ferro- und die andere paramagnetisch und gleichzeitig eine Phase elektrochemisch edler und eine Phase unedler ist, kann somit über die Legierungszusammensetzung, d. h. den Gehalt an Mangan und ggf. anderen vorhandenen Legierungselementen, sowohl das elektrochemische Verhalten, speziell die Degradationsgeschwindigkeit in physiologischer Umgebung, als auch das magnetische Verhalten des Werkstoffs eingestellt werden. Diese Eigenschaft kann somit dafür verwendet werden, biologisch abbaubare Werkstoffe mit steuerbarer Degradationsgeschwindigkeit herzustellen.

Anzumerken ist, dass Mangan für alle biologischen Organismen essentiell ist. Mangelerscheinungen beim Menschen sind jedoch nicht bekannt und selbst hohe Überdosen werden gut verkraftet. Der Tagesbedarf liegt bei 0,4 - 10 mg. Im Tierexperiment wurde Mangan als essentielles Element für die Knochen- und Knorpelbildung identifiziert. Weitere Tieruntersuchungen haben bei Mangan-Mangel Defizite in der Insulinproduktion gezeigt, Veränderungen im Lipoproteinstoffwechsel und Störungen im Metabolismus von Wachstumsfaktoren. Weiterhin ist Mangan möglicherweise ein notwendiger Kofaktor bei der Umwandlung von Präprothrombin zu Prothrombin. Biochemische Untersuchungen haben weiterhin gezeigt, dass Mangan einen Kofaktor für eine Reihe von Enzymen darstellt, unter anderem für die Arginase und die alkalische Phosphatase der Leber sowie für die Pyruvatcarboxylase. Durch Mangan wird auch die Aktivität der Succinatdehydrogenase und der Prolidase sowie einiger Enzyme der Mukopolysaccharidsynthese gesteigert. Dementsprechend ist die Freisetzung von geringen Mengen Mangan im Körper im Zuge der Degradation der Legierung toxikologisch unbedenklich.

Durch weiteres Zulegieren von einem oder mehreren der o. g. Legierungselemente X (Pt, Pd, Ir, Rh, Re, Ru, Os) kann die Korrosionsbeständigkeit von Eisen-Mangan-Legierungen weiter gesenkt werden. Dies wurde beispielhaft am System Fe-Mn-Pd belegt. Hier konnte durch Zulegieren von 0.2 bis 5% Palladium zu Legierungen mit 10 bzw. 20% Mn die Korrosionsgeschwindigkeit, gemessen am Massenverlust einer in SBF ausgelagerten Probe, um mehr als den Faktor 30 gesteigert werden. Alle obig beschriebenen Eigenschaften hinsichtlich der möglichen Einstellung von Phasenanteilen im Gefüge, der magnetischen Eigenschaften bzw. des Korrosionsverhaltens bleiben bei diesen 3- oder Mehrstoffsystemen sinngemäß erhalten. Bevorzugt, insbesondere in Kombination mit der zuvor ausgeführten Legierungsvariante mit einem Mn-Gehalt von 10 bis 20 Gew.%, beträgt der Anteil von X an der Eisenlegierung der Formel (2) 0,01 bis 10 Gew.%. Besonders bevorzugt steht X für Pd.

Ist in einem solchen biologisch abbaubaren Legierungssystem, basierend auf Fe und Mn, die Ausbildung der ferromagnetischen Phase nicht erwünscht, besteht die Möglichkeit dies mit Legierungszusätzen an Kohlenstoff und Stickstoff zu realisieren. Fe-Mn-Legierungen verhalten sich so, dass in Anwesenheit von Kohlenstoff bei wesentlich geringeren Mn-Gehalten ein vollständig kubisch-flächenzentriertes Gefüge erhalten werden kann. Der gleiche Effekt kann auch mit einem Auflegieren mit Stickstoff erreicht werden. Diese Methode ist aus der Herstellung der nickelfreien austenitischen Stähle bekannt. Auch hier wird die Konzentration an Mangan, die zur vollständigen Austenitisierung erforderlich ist, abgesenkt. Eine Zulegierung von Stickstoff oder Kohlenstoff in das System Fe-Mn eröffnet hiermit die Möglichkeit zum Einen die Degradationseigenschaften hinsichtlich eines beschleunigten Abbaus zu optimieren und gleichzeitig das Gefüge hinsichtlich einer möglichst guten Umformbarkeit oder hinsichtlich seiner MR-Kompatibilität zu optimieren. Besonders bevorzugt ist eine Kombination dieser Legierungsvariante mit den zuvor ausgeführten Legierungsvarianten mit einem Mn-Gehalt von 10 bis 20 Gew.% und/oder mit einem X-Anteil von 0,01 bis 10 Gew.%.

In der Eisenlegierung der Formel (3):

Fe-Z (3)

steht Z für ein oder mehrere Elemente, ausgewählt aus der Gruppe Pt, Ir und Os und ein Anteil von Z an der Legierung beträgt 5 bis 30 Gew.%. Insbesondere die Elemente Iridium und Platin verhalten sich grundsätzlich hinsichtlich der Phasenausbildung wie Mangan; auch hier lassen sich bei geeigneter Temperaturführung Duplexgefüge erzeugen. Infolge der sehr positiven Standardpotentiale von Platin und Iridium bilden sich zwei Phasen mit unterschiedlichen elektrochemischen Eigenschaften. Durch Lokalelementbildung zwischen den beiden Phasen wird der Degradationsprozess beschleunigt.

Implantate im Sinne der Erfindung sind über ein chirurgisches oder minimalinvasives Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes, beispielsweise Stents, und Ankerelemente für Elektroden, insbesondere von Schrittmachern oder Defibrillatoren. Das Implantat besteht ganz oder in Teilen aus dem biokorrodierbaren Werkstoff.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird.

Besonders bevorzugt ist das Implantat als Implantat zur Hyperthermiebehandlung ausgebildet, d. h. das Implantat ist zu diesen Zwecken hergerichtet. Insbesondere wird das Implantat zur Tumortherapie durch Radiofrequenz-Ablation eingesetzt bzw. ist entsprechend hergerichtet. Das Implantat ist also so beschaffen, dass es im magnetischen Wechselfeld durch Induktion erwärmt wird. Das Implantat ist so ausgebildet, dass es für die Zwecke der Hyperthermiebehandlung geeignet ist. Dies beinhaltet die Vorgabe einer dem Anwendungsort und Wechselspiel mit dem Erreger angepassten Geometrie.

Hier kommt eine weitere Besonderheit der Legierungssysteme, wie insbesondere Fe-Mn, Fe-Pd, Fe-Ir, Fe-Pt, zum Tragen. Die Eisenlegierungen öffnen das sogenannte γ-Feld, d.h. mit zunehmendem Legierungsgehalt verschiebt sich die Temperatur, bei der sich das α+γ Duplexgefüge mit einem ferro- und einem paramagnetischen Anteil komplett in des paramagnetische γ-Gefüge umwandelt kontinuierlich nach unten. Die α-γ Umwandlungstemperatur von reinem Eisen kann so z. B. legierungstechnisch, je nach Wahl des Systems, bis auf Werte zum Teil unter 100°C abgesenkt werden. Dies wiederum bedeutet, dass die ferromagnetische Phase, die wegen ihrer wesentlich stärkeren Wechselwirkung mit magnetischen Feldern maßgeblich die Erwärmung bewirkt, oberhalb einer bestimmten Temperatur verschwindet und damit die weitere Erwärmung eines solchen Bauteils oder Implantats solange ausbleibt, bis die Temperatur soweit abgefallen ist, dass sich die ferromagnetische α-Phase wieder ausbilden kann. Im Ergebnis bedeutet das, dass die Temperatur nie über einen Grenzwert ansteigt; dieser Grenzwert ist durch die Einstellung der Phasenanteile des Gefüges steuerbar. Werden z. B. hohe Anteile der oben genannten Legierungselemente zulegiert, dann besitzt das resultierende Gefüge eine niedrige Temperatur bei der es komplett paramagnetisch wird und auch im Koexistenzgebiet der beiden Phasen nur einen sehr kleinen Gefügeanteil der ferromagnetischen γ-Phase. Ein solcher Werkstoff wird sich z. B. nur auf vergleichsweise geringe Temperaturen erwärmen lassen und außerdem wegen des ständigen Wärmeabflusses in der resultierenden Temperatur noch einmal deutlich unter der Umwandlungstemperatur liegen. Die kann bei der Anwendung als biologisch abbaubare Legierung zur Herstellung von Implantaten zur Hyperthermiebehandlung dazu verwendet werden, die Temperaturen z. B. bei einem RF-Ablationsprozeß zu begrenzen und dadurch zu große Gewebeschäden durch zu hohe Temperaturen zu vermeiden. In einem magnetischen Wechselfeld erfahren demnach die Implantate zur Hyperthermiebehandlung aus der erfindungsgemäßen Eisenlegierung eine definierte Erwärmung. Damit besteht erstmalig die Möglichkeit, biologisch abbaubare Implantate mit definierbarer Degradationskinetik zur Hyperthermiebehandlung, z. B. im Zuge einer Tumortherapie, einzusetzen.

Ein weiterer Aspekt der Erfindung ist damit die Verwendung einer biokorrodierbaren Eisenlegierung der Formeln (2) und (3) zur Herstellung eines Implantats, das für die Hyperthermiebehandlung ausgebildet ist.

Neben der Möglichkeit der Erwärmung im elektromagnetischen Wechselfeld besitzen zumindest partiell ferromagnetische Eisenlegierungen der Formeln (1) bis (3), also solche mit einer ausreichend breiten Hysteresekurve, nach einer Ausrichtung der magnetischen Momente ein permanentes magnetisches Moment. Diese Eigenschaft kann dazu genutzt werden Implantate herzustellen, die unter Nutzung magnetischer Anziehungskräfte Gewebe gegen Gewebe fixieren können, solange bis sie abgebaut sind. Ein weiterer Aspekt der Erfindung ist somit die Verwendung einer biokorrodierbaren Eisenlegierung der Formeln (1) bis (3) zur Herstellung eines Implantats, das zur temporären Fixierung von Gewebe ausgebildet ist.

Eine weitere Besonderheit der Legierungen zeigt sich bei plastischer Verformung. Werden Legierungen der Gruppe Fe-Mn-X, beispielsweise Fe-Mn-Pd, plastisch verformt, können zwei Besonderheiten beobachtet werden.

Zum Einen ist die sogenannte Verfestigung dieser Legierungen extrem hoch, d. h. die Legierungen lassen sich bei relativ geringen Spannungen (Kräften) plastisch verformen; während der Verformung steigt die Festigkeit der Legierung kontinuierlich stark an. Das Verhältnis von Streckgrenze (Beginn der plastischen Verformung) und maximaler Festigkeit ist dabei ungewöhnlich hoch. So wurde bei ersten Versuchen bei der Legierung FeMn15Pd1 eine Streckgrenze im Bereich von 350 MPa und eine maximale Festigkeit (Zugfestigkeit) im Bereich von 900 MPa gefunden. Damit liegt die Festigkeit dieser Legierungen erheblich über der von chirurgischem Edelstahl (316L) und erreicht fast die Festigkeitswerte von CoCr-Legierungen wie L605 oder MP35N, den höchstfesten Legierungen, die für Stents verwendet werden.

Zum Anderen besteht eine weitere Besonderheit bei der Verformung dieser Legierungen darin, dass bei der Verformung eine dehnungsinduzierte Phasenumwandlung stattfindet; das bedeutet, dass sich in dem zweiphasigen Gefüge zumindest ein Teil einer Phase in die zweite Phase des Duplexgefüges umwandelt. Im vorliegenden Fall geschieht dies gerade so, dass bei der Verformung der Anteil der ferromagnetischen Phase zunimmt.

Werden diese Legierungen daher für Implantate verwendet, die plastisch verformt werden, wie insbesondere ballonexpandierbare Stents, hat die starke Verfestigung bei der plastischen Verformung verschiedene Vorteile:
So erreichen Stents, bedingt durch den hohen Festigkeitsanstieg des Werkstoffs, eine hohe Radialfestigkeit. Bei einer vorgegeben Radialfestigkeit, können ferner die Dicke der Profile der Struktur, die so genannten Streben (Struts), wesentlich filigraner ausgelegt werden. Diese filigranere Auslegung von Streben hat in der Vergangenheit, bei der Verwendung von CoCr-Legierungen, bereits zu signifikant verbesserten klinischen Ergebnissen geführt. Da die Festigkeit der beschriebenen neuen Legierungen jetzt bereits fast das Niveau der CoCr-Legierungen erreicht, kann mit vergleichbaren Strebendicken und Vorteilen für das Implantat gerechnet werden.

Eine Minimierung der Strebendicken führt bei einem abbaubaren Implantat weiterhin dazu, dass weniger Material in den Körper eingebracht werden muss, was die Gefahr von bioinkompatiblen Reaktionen vermindert und gleichzeitig auch zu einer verkürzten Abbauperiode führt.

Schließlich ist ein zusätzlicher Effekt eine Verringerung des Gesamtprofils des SDS (Stent Delivery System) mit dem montierten Stent.

Die Eigenschaft, dass bei einer plastischen Verformung solcher Legierungen der ferromagnetische Anteil des Gefüges zunimmt, kann in dem Moment genutzt werden, wenn das Implantat, wie oben bereits beschrieben, in einem elektromagnetischen Wechselfeld erwärmt werden soll. In diesem Fall ist die Wechselwirkung mit dem Feld stärker und es lässt sich mit einem geringeren Werkstoffvolumen ein gleichstarker Effekt erreichen.

Das Implantat aus der biokorrodierbaren Eisenlegierung ist demnach insbesondere ein Stent für Blutgefäße, Gallengang, Harnröhre, Speiseröhre etc.; d. h. ein Stütz- oder Verbindungsimplantat für alle Gefäße, Gangsysteme oder Hohlraumverbindungen im menschlichen Körper.

Weiterhin ist das Implantat aus der biokorrodierbaren Eisenlegierung insbesondere ein Clip zum Verschließen von abgetrennten Blutgefäßen. Zum Beispiel ein v-förmiger Clip, mit dem ein abgetrenntes Gefäß verschlossen wird, in dem der Clip mit einer Zange am Gefäßende so zusammengequetscht /plastisch deformiert wird, dass er das Gefäßende verschließt, so dass der Blutfluss zum Stillstand kommt und das Blut thrombosiert. Außerdem kann das Implantat aus der biokorrodierbaren Eisenlegierung insbesondere ein Implantat sein, das dazu verwendet wird, Gefäße, in die eine Kanüle oder ein Katheter mit größerem Durchmesser in das Gefäßsystem temporär eingeführt worden war, nach Entfernen des temporären Implantats, wieder sicher zu verschließen, um Blutungen an dieser Stelle zu vermeiden. In diesem Fall hat das Implantat typischerweise die Form einer Klammer, die mittels eines Applikationssystems implantiert wird und bei der Krallen oder Spitzen die Gefäßwand um die zu schließende Stelle herum greifen und die Öffnung zusammen pressen.

Schließlich ist das Implantat aus der biokorrodierbaren Eisenlegierung insbesondere ein Okkluder. Ein Okkluder ist ein minimalinvasiv applizierbares Fixierungssystem, mit dem z.B. ein Septumdefekt (PFO) so lange fixiert werden kann bis die Herzscheidewand verwachsen und der Defekt natürlich verschlossen wird. Danach kann das Implantat biologisch abgebaut werden. Dabei wird z. B. das Implantat so ausgeführt, dass ein langes röhrenförmiges Gebilde durch eine Zugkraft vor und hinter dem Defekt zusammengestaucht und plastisch verformt wird, so dass sich auf beiden Seiten des Defekts eine Schirmform ausbildet, die beide Teile der offenen Herzscheidewand zusammenpresst.

### Kurzbeschreibung der Zeichnungen

Die Erfindung wird nachfolgend anhand eines Ausführungsbeispiels und den dazugehörigen Zeichnungen näher erläutert. Es zeigen:
- Figur 1: den Fe-reichen Ausschnitt aus einem Fe-P Zustandsdiagramm;
- Figur 2: Probekörper nach dem Abguss im Vakuum-Induktionsofen;
- Figur 3: ein Gefüge der Probekörper im Gusszustand;
- Figur 4: ein Gefüge der Probekörper im homogenisierten Zustand;
- Figur 5: Bode-Plots der untersuchten Legierungen;
- Figur 6: ein Zustandsdiagramm Fe-Mn;
- Figur 7: Gefügebilder der Legierungen;
- Figur 8: Bode-Plots der untersuchten Legierungen;
- Figur 9: ein reales Zustandsdiagramm Fe-Mn;
- Figur 10: Gefügebilder von Reineisen;
- Figur 11: Gefügebilder der Legierungen Fe-10Mn-xPd;
- Figur 12: Gefügebilder der Legierungen Fe-20Mn-xPd;
- Figur 13: Gefügebilder der Legierungen Fe-10Mn-xPd nach Lösungsglühung;
- Figur 14: Gefügebilder der Legierungen Fe-20Mn-xPd nach Lösungsglühung;
- Figur 15: Härtewerte der Legierungen im Zustand 1100°C/2h + 700°C/1h;
- Figur 16: Bode-Plots der untersuchten Pd-freien Legierungen (1100°C/2h + + 700°C/1h);
- Figur 17: Bode-Plots der untersuchten Legierungen mit 10% Mn (1100°C/2h + 700°C/1h);
- Figur 18: Bode-Plots der untersuchten Legierungen mit 20% Mn (1100°C/2h + 700°C/1h);
- Figur 19: Masseverlust in SBF (1100°C/10h/H₂O + 700°C/5h);
- Figur 20: Masseverlust in SBF (1100°C/10h/langsam + 700°C/5h) und
- Figur 21: einen Zugversuch an FeMnPd-Legierung.

### System Fe-P

### Legierungsherstellung

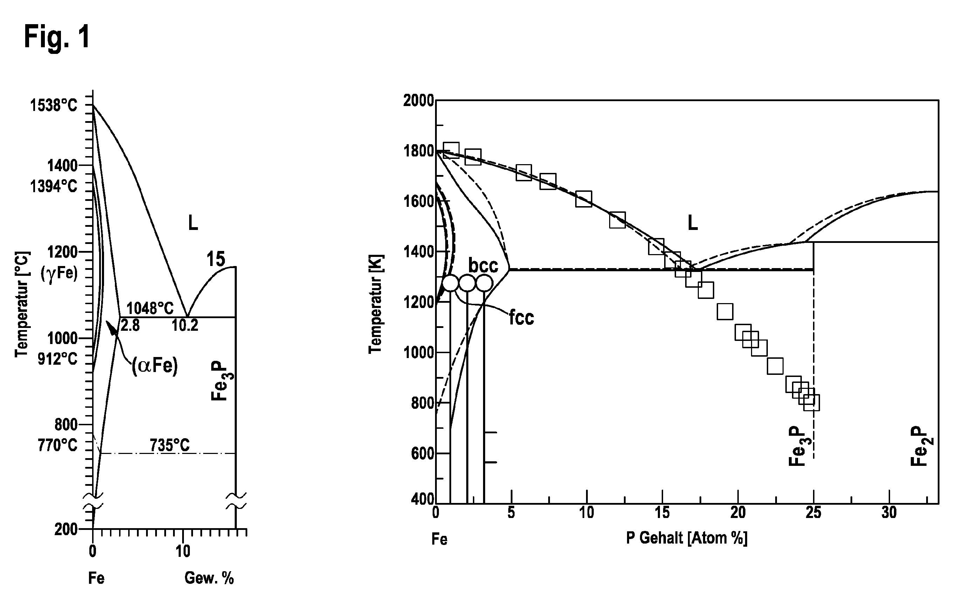

Fig. 1 zeigt den Fe-reichen Ausschnitt aus einem Fe-P Zustandsdiagramm. Dem Zustandsdiagramm lassen sich folgende, für die Zwecke der Erfindung bedeutsame Merkmale entnehmen:
1) P schnürt das γ-Gebiet stark ein, P ist also ein starker Ferritstabilisator;
2) Mit dem Eisenphosphid Fe₃P bildet sich ein tiefschmelzendes Eutektikum (Tₑ=1048°C);
3) Die maximale Löslichkeit beträgt nur 2.8 Gew.%, entsprechend 4.9 Atom%.

Bei der Probenerschmelzung wurden entsprechend folgende Punkte beachtet:
1) Die Zusammensetzung sollte innerhalb des einphasigen Ferritbereiches zu liegen kommen, so dass kein stark versprödendes Fe₃P-Eutektikum gebildet wird, wenn der Werkstoff später plastisch verformt werden soll;
2) Da bei der Erschmelzung die Legierung im Ungleichgewicht vorliegt, sollte eine Homogenisierungsglühung durchgeführt werden; demzufolge sollte die Zusammensetzung nicht zu nahe an der maximalen Löslichkeit liegen, da sonst das Temperaturfenster für die Glühbehandlung zu eng wird;
3) Um den P-Einfluss auf Struktur, mechanische und elektrochemische Eigenschaften studieren zu können, sollte eine Legierungsserie mit mindestens drei P-Leveln erschmolzen werden.

Gewählt wurde eine Legierungsserie mit 1, 2 und 3 At%, entsprechend 0.56, 1.12 und 1.69 Gew.% (siehe rechtes Teilbild in Fig. 1).

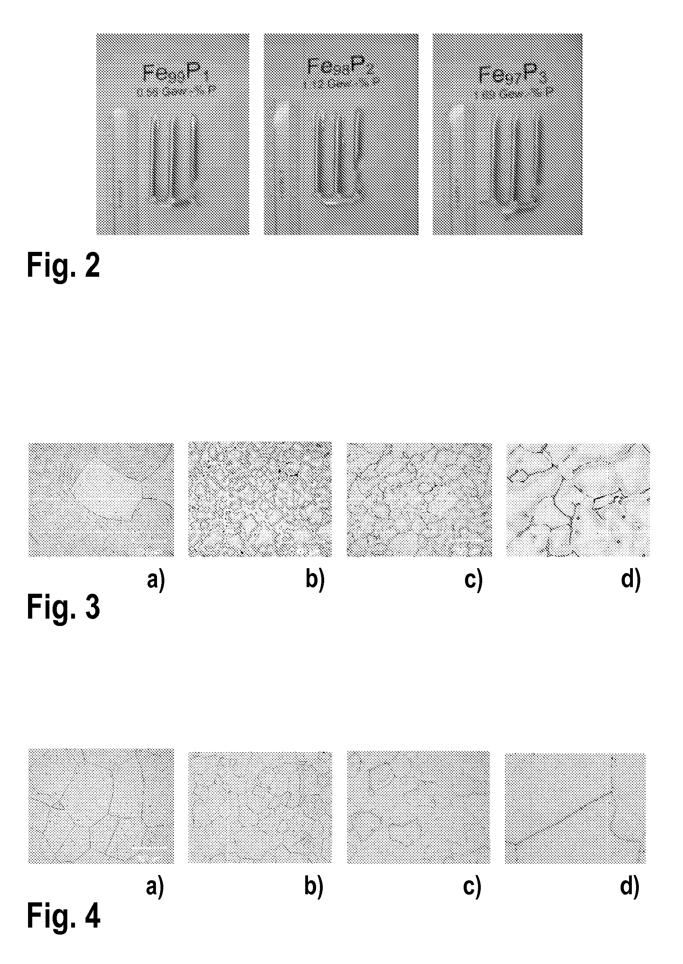

Die Erschmelzung der Legierungen erfolgte im Vakuum-Induktionsofen mit einem Chargengewicht von ca. 400 g. Phosphor wurde in Form einer Eisen-Phosphid-Vorlegierung zulegiert. Der Probenabguss wurde im Vakuum-Induktionsofen durchgeführt. Dazu wurde eine mehrteilige Cu-Kokille verwendet, die den Abguss von Probestäben möglich macht (Fig. 2).

### Metallographische Charakterisierung

Fig. 3 zeigt Gefüge der Probekörper im Gusszustand Fig. 3a: Fe₉₉P₁, 3b: Fe₉₈P₂, 3c: Fe₉₇P₃, 3d: Fe₉₇P₃, Ausschnitt um Faktor 4 vergrößert. Man erkennt die ausgeprägte Seigerungsneigung. Während das Gefüge der Legierung mit 1 At% P einphasig mit groben Korn vorliegt, weisen die Legierungen mit 2 und 3 At% P aufgrund der Ungleichgewichtserstarrung schon eutektische Anteile auf. Diese Gefügebereiche wirken stark versprödend; so war es beispielsweise möglich, die in Abbildung 2 gezeigten unvollständig gefüllten rechten Probestäbe an den dünnen Stellen "per Hand" zu brechen, und zwar mit dem typischen Bruchbild eines interkristallinen Sprödbruchs.

Um die Testlegierungen in einen gleichgewichtsnahen Zustand überzuführen wurde eine Lösungsglühung bei 1000°C (1270K) durchgeführt (siehe Zustandspunkte in Abbildung 1 rechts). Entsprechend dem Phasendiagramm sollten sich einphasig-ferritische Gefüge einstellen lassen. Abbildung 4 zeigt die Gefüge der untersuchten Legierungen nach einer 2-stündigen Glühung mit nachfolgender Wasserabschreckung.

Fig. 4 zeigt Gefüge der Probekörper im homogenisierten Zustand - 100°C/2h/Wasser Fig. 4a: Fe₉₉P₁, 4b: Fe₉₈P₂, 4c: Fe₉₇P₃, 4d: Fe₉₇P₃, Ausschnitt um Faktor 7 vergrößert. Man erkennt den einphasigen Zustand; die eutektischen Phasenanteile haben sich vollständig aufgelöst.

Härtemessungen an den Schliffproben zeigen den signifikanten Härtungseffekt durch das Legierungselement Phosphor. Die Härte steigt von HV10 = 95 ± 15 bei Reineisen auf HV10 = 160 ± 10 bei Fe₉₉P₁, HV10 = 230 ± 10 bei Fe₉₈P₂ und HV10 = 290 ± 10 bei Fe₉₇P₃. Dieser ausgeprägte Mischkristallhärtungseffekt ist verbunden mit einer Versprödung.

Vorzugsweise beträgt ein Anteil von P an der Eisenlegierung der Formel (1) 0,01 bis 1,2 Gew.%.

### Korrosionsverhalten - Elektrochemische Impedanzspektroskopie (EIS) und Degradationsversuche

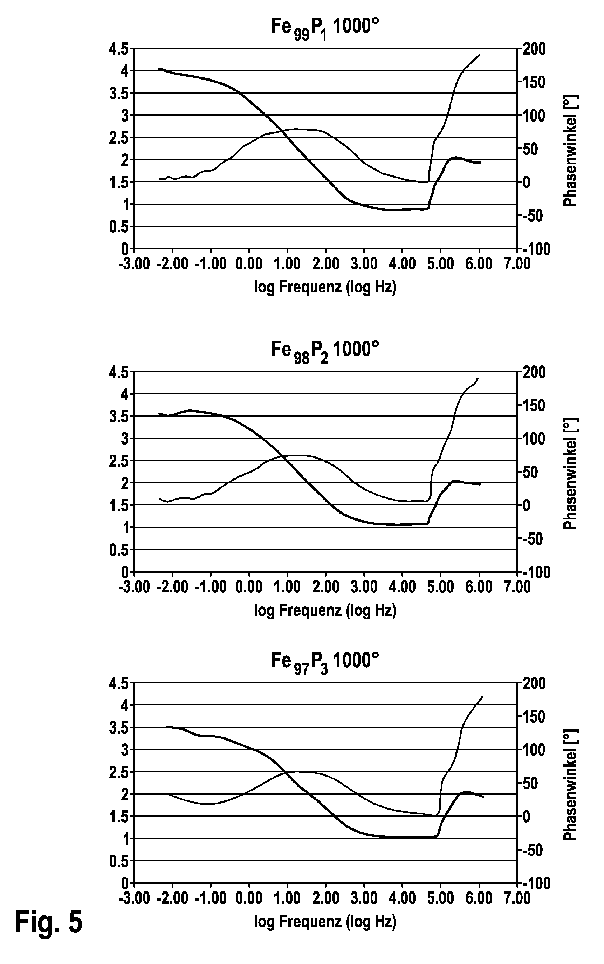

Die elektrochemischen Eigenschaften von Reineisen bzw. den Legierungen wurde mit Hilfe von Impedanzspektroskopie unter folgenden Bedingungen bestimmt: Prüftemperatur: 22.9°C; Prüfmedium: SBF, pH 8.14; Referenzelektrode: Hg-Hg-Cl; Gegenelektrode: Platin; Testfläche Ø 7.4 mm. Die Bode-Plots sind in Fig. 5 dargestellt.

Die EIS Ergebnisse zeigen, dass durch das Zulegieren von Phosphor die Korrosionsempfindlichkeit zunimmt. Eine Abschätzung mit den Werten der Impedanz bei geringen Frequenzen ergibt für die Legierungen mit 1 und 2 At% P einen um etwa den Faktor 3 geringeren Wert.

### System Fe-Mn (nicht zur Erfindung gehörend)

### Legierungsherstellung

Im Labormaßstab wurden Probekörper der folgenden Zusammensetzung erschmolzen:
- Reineisen (Fe);
- Reineisen + 10 Gew.% Mangan (Fe-10Mn); und
- Reineisen + 20 Gew.% Mangan (Fe-20Mn).

Die Synthese erfolgte in einem Lichtbogenofen unter Argon-Atmosphäre. Das Chargengewicht betrug etwa 7 g; dementsprechend war das Legierungsprodukt ein ellipsoidaler Probekörper mit ca. 10 bis 12 mm Durchmesser.

### Metallographische Charakterisierung

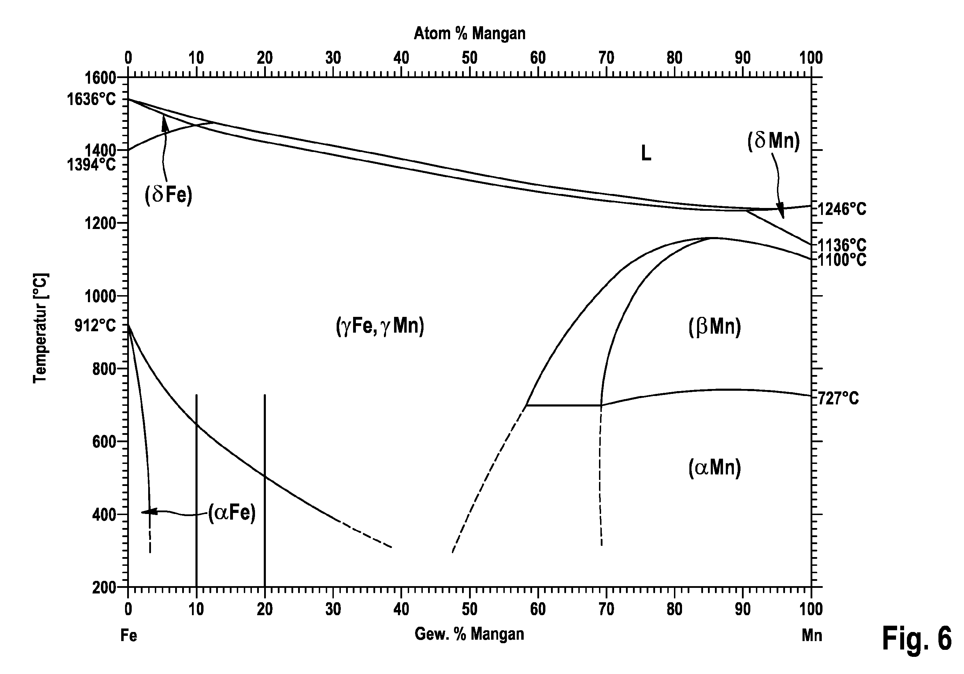

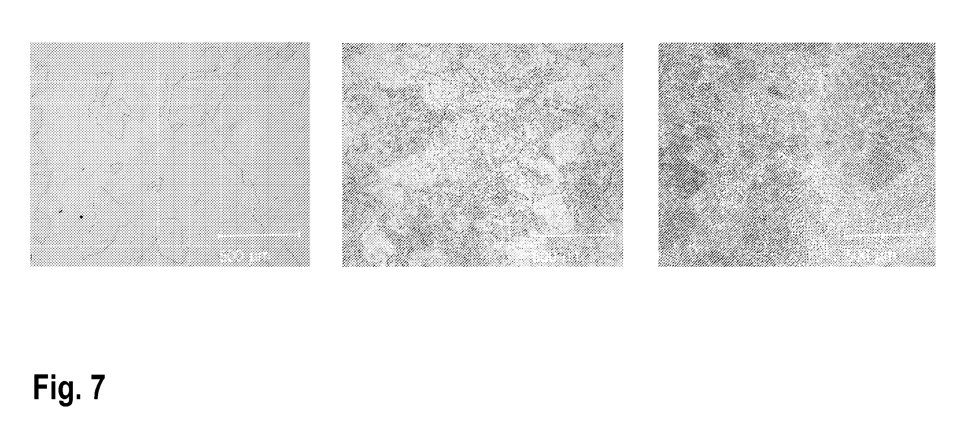

Fig. 6 zeigt das binäre System Fe-Mn mit Kennzeichnungslinien für die gewählten Zusammensetzungen. Für die Legierungen mit 10 bzw. 20 Gew.% Mangan ist ein zweiphasiges α+γ Gefüge zu erwarten. Die Gefügebilder in Fig. 7 bestätigen die Vermutung und zeigen Gefügebilder der Legierungen Fe (links), Fe-10Mn (Mitte) und Fe-20Mn (rechts). Man erkennt die beiden Phasen α-Fe und γ-Fe. Anzumerken ist jedoch, dass es sich dabei um nicht vollständig im Gleichgewicht befindliche Gussstrukturen handelt. Durch Homogenisierungsglühung mit nachfolgender Umformung können jedoch rekristallisierte Strukturen mit geringerer Korngröße erzeugt werden.

Härtemessungen an den Schliffproben zeigen den signifikanten Härtungseffekt durch das Legierungselement Mangan. Die Härte steigt von HV10 = 95 ± 15 bei Reineisen auf HV10 = 220 ± 10 bei Fe-10Mn und HV10 = 230 ± 10 bei Fe-20Mn. Aufgrund der kubischflächenzentrierten γ-Phase ist bei den Legierungen mit 10 bzw. 20% Mn auch mit einer Duktilitätserhöhung zu rechnen.

### Korrosionsverhalten - Elektrochemische Impedanzspektroskopie (EIS)

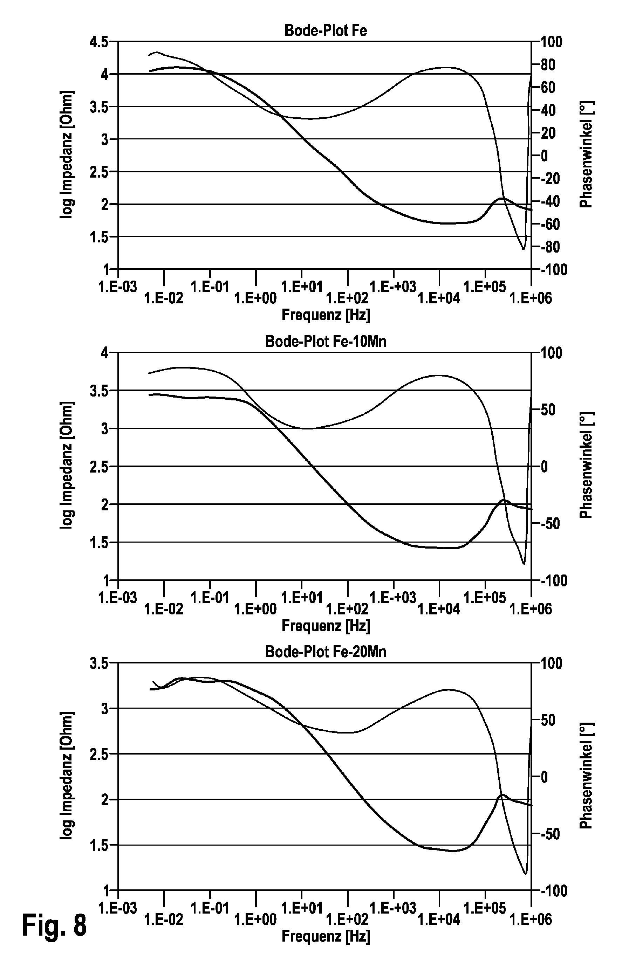

Die elektrochemischen Eigenschaften von Reineisen bzw. den Legierungen wurden mit Hilfe von Impedanzspektroskopie unter folgenden Bedingungen bestimmt: Prüftemperatur: 22.9°C; Prüfmedium: SBF, pH 8.14; Referenzelektrode: Hg-Hg-Cl; Gegenelektrode: Platin; Testfläche Ø 7.4 mm. Die Bode-Plots sind in Fig. 8 dargestellt.

Die EIS Ergebnisse zeigen, dass durch das Zulegieren von Mangan der Korrosionsempfindlichkeit signifikant zunimmt. Eine Abschätzung mit den Werten der Impedanz bei geringen Frequenzen ergibt für die Legierung mit 10% Mangan eine um den Faktor 3.5 geringeren Wert, für die Legierung mit 20% Mangan ergibt sich eine Abschwächung um etwa den Faktor 6.

### System Fe-Mn-X

Durch Zulegieren von "edlen" Elementen X werden fein verteilte "edle" Ausscheidungen gebildet, die als kathodische Stellen wirken und so zu verstärkter galvanischer Korrosion führen. Bei der Selektion der Elemente X sind verschiedene Aspekte zu beachten: i) die Elemente sollten eine beschränkte Löslichkeit haben, d. h. Ausscheidungen bilden; ii) die sich bildenden Zweitphasen, in der Regel Intermetallische Phasen (IMP), sollen einen möglichst hohen Gehalt am edlen Metall haben, so dass sie auch stark kathodisch wirken; iii) die Biokompatibilität sollte gewährleistet sein.

Das binäre System der Elemente Fe und Pd lässt Pd als besonders geeignet erscheinen. Die möglichen IMP's enthalten alle mindestens 50 At% Pd, während beim Pt auch IMP's mit geringerem Pt-Gehalt gebildet werden. Bei Ir und Rh sind die binären Systeme mit Mn zu wenig gut dokumentiert um vergleichbare Aussagen machen zu können.

Wie aus dem binären System Fe-Mn entnommen werden kann, ist die γ/α Umwandlung mit erheblichen Konzentrationsänderungen verbunden. Da sich mit steigendem Mn-Gehalt die Umwandlung zu tieferen Temperaturen verschiebt, wird die Diffusion stark erschwert. Ab einem Gehalt von 5 Gew.% Mn wandelt sich der Austenit bei üblicher Abkühlungsgeschwindigkeit nicht unter Konzentrationsausgleich in Ferrit um, sondern es erfolgt eine diffusionslose Schiebungsumwandlung in kubischen Martensit.

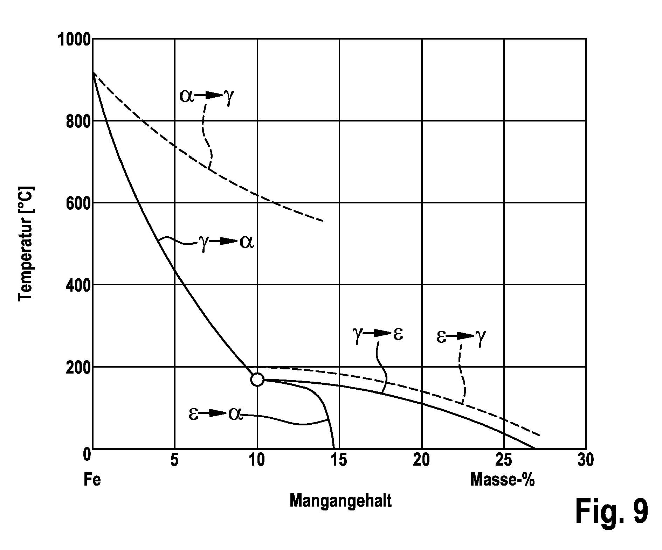

Fig. 9 ist das Realdiagramm von Fe-Mn Legierungen zu entnehmen. Die Umwandlung zu Martensit erstreckt sich über ein größeres Temperaturintervall. Bei einer nachfolgenden Erwärmung beginnt die Rückumwandlung, sobald die bei wesentlich höheren Temperaturen liegende α → γ Linie erreicht wird. Diese Erscheinung nennt man Irreversibilität und tritt bei Fe-Mn Legierungen bis etwa 10% Mn auf. Zwischen 10 und 14.5% Mn entsteht während der Abkühlung zunächst hexagonaler ε- Martensit, der dann zu α- Martensit umwandelt. Beim Wiedererwärmen wandelt sich der ε- Martensit bereits zwischen 200 - 300°C zu Austenit zurück, der α- Martensit aber erst bei deutlich höheren Temperaturen. In Legierungen mit <14.5% Mn erfolgt während der Abkühlung nur die γ → ε Umwandlung, die aber unvollständig ist und stets große Restaustenitmengen vorliegen. Beim Wiedererwärmen erfolgt die Rückumwandlung bei nur wenig höheren Temperaturen. Die γ → ε Umwandlung kann durch rasches Abkühlen vollständig unterdrückt werden. Das Umwandlungsverhalten kann also dahingehend interpretiert werden, dass bei einer Temperatur von >600°C bei Legierungen mit >10% Mn immer austenitisches Gefüge vorliegt. Aufgrund der im Austenit gegenüber Ferrit bzw. Martensit deutlich geringeren Diffusion ist bei einer entsprechenden Glühbehandlung mit "feiner" Ausscheidung von Pdhaltigen IMP's zu rechnen.

### Legierungsherstellung

Folgende Legierungen wurden hergestellt:
- Reineisen;
- Fe-10 Gew.% Mn mit jeweils 0%, 0.2%, 1% und 5 Gew.% Pd; und
- Fe-20 Gew.% Mn mit jeweils 0%, 0.2%, 1% und 5 Gew.% Pd.

Die verwendeten Ausgangsmaterialien waren:
- Eisenpulver: Ø < 200µm, Reinheit > 99%
- Manganpulver: Ø < 200µm, Reinheit > 99.9%
- Palladium, Reinheit > 99.99%

Jeweils 50 bis 60 g Legierung wurden produziert. Dazu wurde eine Ausgangsmischung in einem Al₂O₃-Tiegel im Vakuum-Induktionsofen bei 0.3 bar Argon rasch aufgeschmolzen und in Stiftform (Durchmesser ca. 10.5 mm) abgegossen. Die Stifte wurden anschließend in ca. 3 mm dicke Scheiben geschnitten.

### Wärmebehandlungen

Für die Wärmebehandlungen wurden die gereinigten Probenscheiben bei 0.3 bar Argon in Quarzglasrohre eingeschweißt. Die Behandlungen erfolgten im vorgeheizten Glühofen, die Proben wurden (wo nicht anders erwähnt) direkt aus dem Ofen kommend im Wasser abgeschreckt. Dabei wurden die Quarzglasrohre im Wasser zerdrückt, so dass die Proben im direkten Kontakt zum Wasser abgeschreckt wurden. Folgende Wärmebehandlungen wurden durchgeführt:
- 2 h bei 1100°C
- 2 h bei 1100°C, 1 h bei 700°C
- 10 h bei 1100°C
- 10 h bei 1100°C, 5 h bei 700°C
- 10 h bei 1100°C, 5 h bei 600°C
- 10 h bei 1100°C und langsames Abkühlen (100°C/h) bis Raumtemperatur (RT)
- 10 h bei 1100°C und langsames Abkühlen (100°C/h) bis Raumtemperatur (RT), 5 h bei 700°C
- 10 h bei 1100°C und langsames Abkühlen (100°C/h) bis Raumtemperatur (RT), 5 h bei 600°C

### Lichtmikroskopische Untersuchungen

Die geschliffenen und polierten Proben wurden unter dem Lichtmikroskop untersucht. Zur Sichtbarmachung des Gefüges wurden folgende Ätzmittel verwendet:
- Fe m3 für Legierungen mit 20% Mangan: 100 ml Stammlösung Klemm + 2 g Kaliumdisulfit; Stammlösung Klemm: 300 ml dest. Wasser (40°C) +1000 g Natriumthiosulfat
- Für Fe und Fe-10%Mn-Varianten wurde Nital als Ätzmittel verwendet.

### Impedanzmessungen

Die Impedanzmessung erlaubt eine Einschätzung des Korrosionsverhaltens: Tiefe Impedanzen (bei tiefen Messfrequenzen) deuten auf eine geringe Korrosionsresistenz hin. Die Messungen wurden mit einem Impedanzspektrometer des Typs AUTOLAB PGSTAT302 durchgeführt.

Prüfmedium: SBF. Referenzelektrode: Hg-Hg-Cl. Gegenelektrode: Platin. Prüffläche der Proben: 72 mm².

Der Rand der in Bakelit eingebetteten Proben wurde vor der Messung mit Silikon abgedichtet, um Spaltkorrosion zwischen Probe und Bakelit zu verhindern.

### Degradationsversuche

Es wurden zwei verschiedene Versuchsvarianten verwendet. Für den ersten wurden die Probenscheiben durchbohrt (Ø 3 mm) und an einem Polymerfaden befestigt ins SBF eingetaucht. Für die zweite Variante wurden die Proben mit Hilfe kleiner Polymer-Probenhalter befestigt und so ins SBF eingetaucht. Als Gefäß wurden abgedeckte 3 I Bechergläser verwendet. Das SBF wurde während der Degradation von einem Magnetrührer umgewälzt. Der Fortschritt der Degradation wurde über den Gewichtsverlust der Proben bestimmt. Dazu wurden die Proben aus dem SBF entfernt, zuerst in destilliertem Wasser, dann in Ethanol mit Ultraschall gereinigt und unter Pressluft getrocknet.

### Mikrostruktur

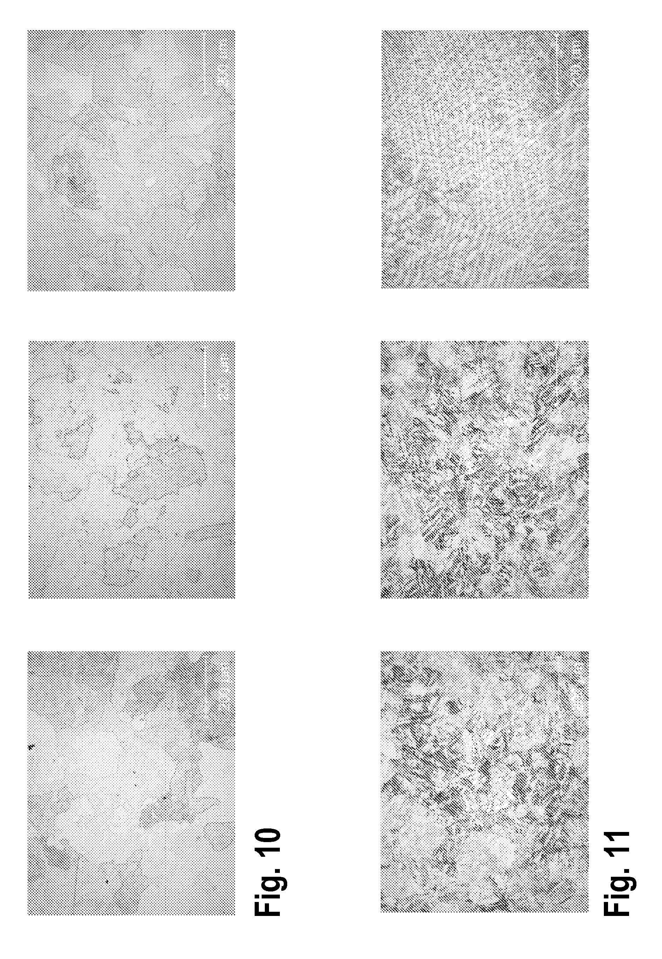

Fig. 10 zeigt Gefügebilder von Reineisen (links: 1100°/2h; Mitte: 1100°/10h; rechts: 1100°/2h+700°C/1h). Es ist eine ferritische Struktur zu erkennen.

Fig. 11 zeigt Gefügebilder der Legierungen Fe-10Mn-xPd (1100°C/2h; links: x=0; Mitte: x=1.0; rechts: x=5). Es liegt α-Martensit vor und es ist eine starke Pd-Seigerung bei 5% Pd zu erkennen.

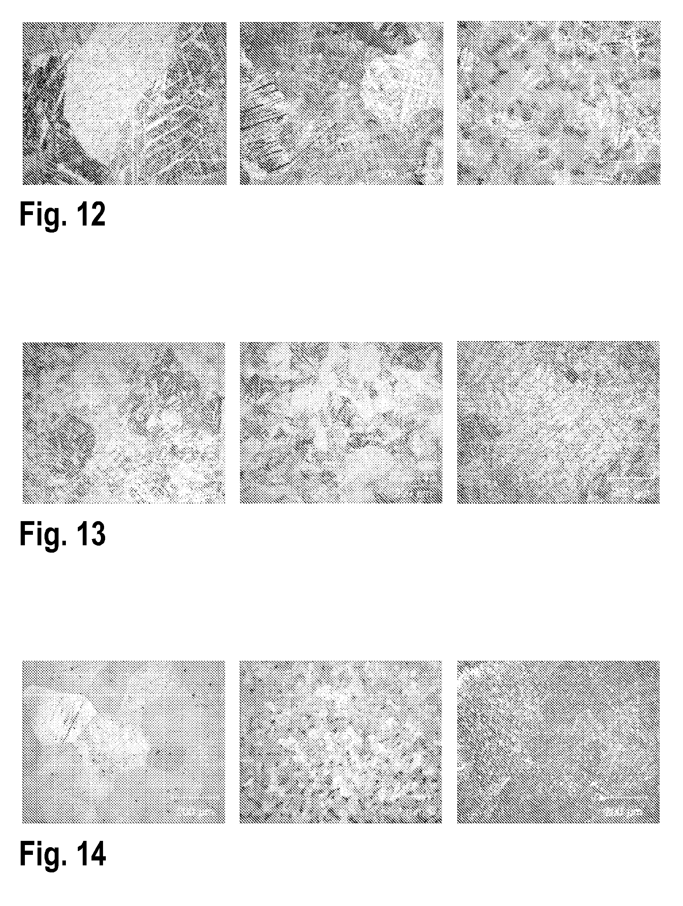

Fig. 12 zeigt Gefügebilder der Legierungen Fe-20Mn-xPd (1100°C/2h; links: x=0; Mitte: x=1.0; rechts: x=5) Es liegt ε-Martensit vor und es ist eine starke Pd-Seigerung bei 1% und 5% Pd zu erkennen.

Fig. 13 zeigt Gefügebilder der Legierungen Fe-10Mn-xPd (1100°C/10h+700°C/1h; links: x=0; Mitte: x=1.0; rechts: x=5). Es liegt α-Martensit vor und es ist eine Pd-Seigerung bei 5%Pd zu erkennen.

Fig. 14 zeigt Gefügebilder der Legierungen Fe-20Mn-xPd (1100°C/10h+700°C/1h; links: x=0; Mitte: x=1.0; rechts: x=5). Es liegt Austenit und ε-Martensit vor und es ist eine Pd-Seigerung bei 5%Pd zu erkennen.

Die Abbildungen der Figuren 10 bis 12 illustrieren beispielhaft die Gefügeeinstellung in Abhängigkeit von Zusammensetzung im Zustand 2 h lösungsgeglüht und abgeschreckt. Auffallend ist die starke Seigerungsneigung. Durch eine längere Lösungsglühung wurde versucht dies auszugleichen. Die Abbildungen der Figuren 13 und 14 zeigen die Gefüge nach dieser längeren Lösungsglühung. Eine Knetbehandlung kann zum Ausgleich der Seigerungen durchgeführt werden. Auffallend ist das unterschiedliche Ätzverhalten, das schon auf unterschiedliche chemische Eigenschaften hinweist.

Zu betonen ist, dass nach der Auslagerung bei 600 bzw. 700°C keine Ausscheidungsbildung mit dem Lichtmikroskop erkennbar war; daraus ist zu schließen, dass sich Ausscheidungen einer Größe von deutlich kleiner als 1 µm gebildet haben.

### Härtemessung

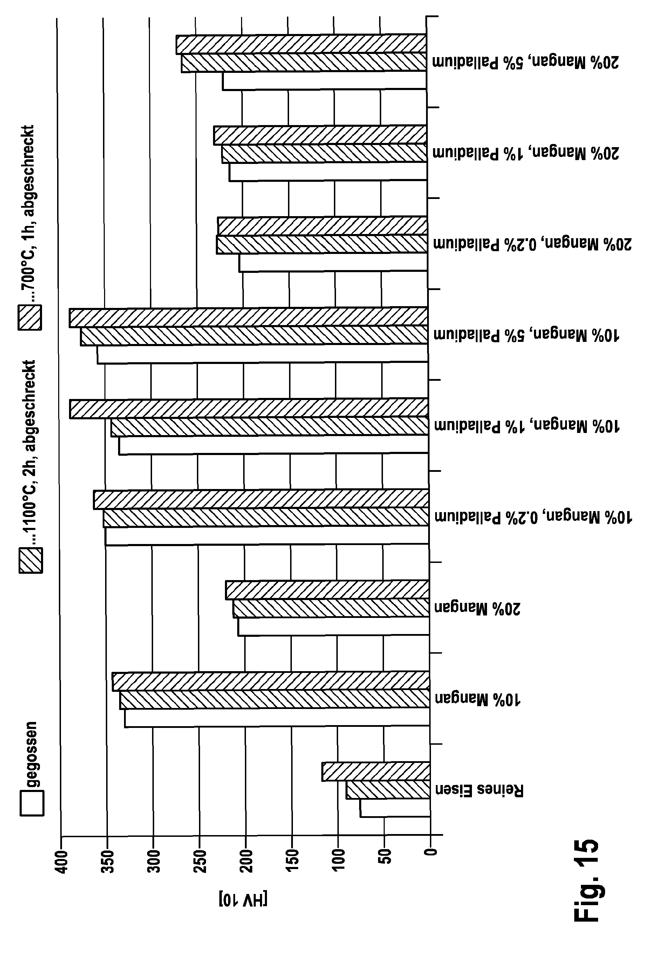

Fig. 15 zeigt Härtewerte der Legierungen im Zustand 1100°C/2h + 700°C/1h. Wie vermutet zeigt sich, dass vor allem der Mangangehalt bzw. das daraus resultierende Gefüge entscheidend ist für die Härte. Das ferritische Gefüge (reines Eisen) ist das weichste, gefolgt vom austenitischen (20% Mangan) und dem martensitischen (10% Mangan).

### Ferrit- und Martensitgehalt

Die Tabelle 1 zeigt die gemessenen Werte für den Ferrit/Martensitgehalt. Die Daten decken sich gut mit den Erkenntnissen aus den Gefügebildern und den Härtemessungen: Das Gefüge ist weitgehend vom Mangangehalt bestimmt. Reines Eisen ist ferritisch (stets Werte von annähernd 100% Ferrit), Legierungen mit 20% Mangan sind austenitisch (stets Werte von <4% Ferrit bzw. "keine Messung" weil reiner Austenit). Auffallend sind aber die großen Unterschiede der gemessen Werte der Legierungen mit 10% Mangan. Sie können durch die verschiedenen Wärmebehandlungen deutlich zu- aber auch abnehmen, was einer Ab- bzw. Zunahme des Austenitgehalts entspricht.

**Tabelle 1: Ferrit-/Martensitgehalt in Prozent.**

| | *1100°C, 2h, abgeschreckt* | | *1100°C, 10h, abgeschreckt* | | |
|---|---|---|---|---|---|
| | - | *+ 700°C, 1h, abgeschreckt* | - | *+ 600°C, 5h, abgeschreckt* | *+ 700°C, 5h, abgeschreckt* |
| *Reines Eisen* | 97.9 | 100 | 100 | 100 | 100 |
| *10% Mn* | 75.8 | 81.9 | - | - | - |
| *20% Mn* | 0.16 | <1 | <1 | 3.3 | 3.6 |
| *10% Mn, 0.2% Pd* | 84.7 | 60.9 | 78.3 | 85.3 | 85.6 |
| *10% Mn, 1% Pd* | 86.8 | 63.4 | 86.7 | 83.5 | 83.1 |
| *10% Mn, 5% Pd* | 82.4 | 76.8 | 90.3 | 77.5 | 79.1 |
| *20% Mn, 0.2% Pd* | 0.25 | <1 | <1 | <1 | <1 |
| *20% Mn, 1% Pd* | k.M. | <1 | <1 | <1 | <1 |
| *20% Mn, 5% Pd* | k.M. | <1 | 2.9 | 0.48 | 0.6 |

### Korrosionsverhalten - Elektrochemische Impedanzspektroskopie (EIS)

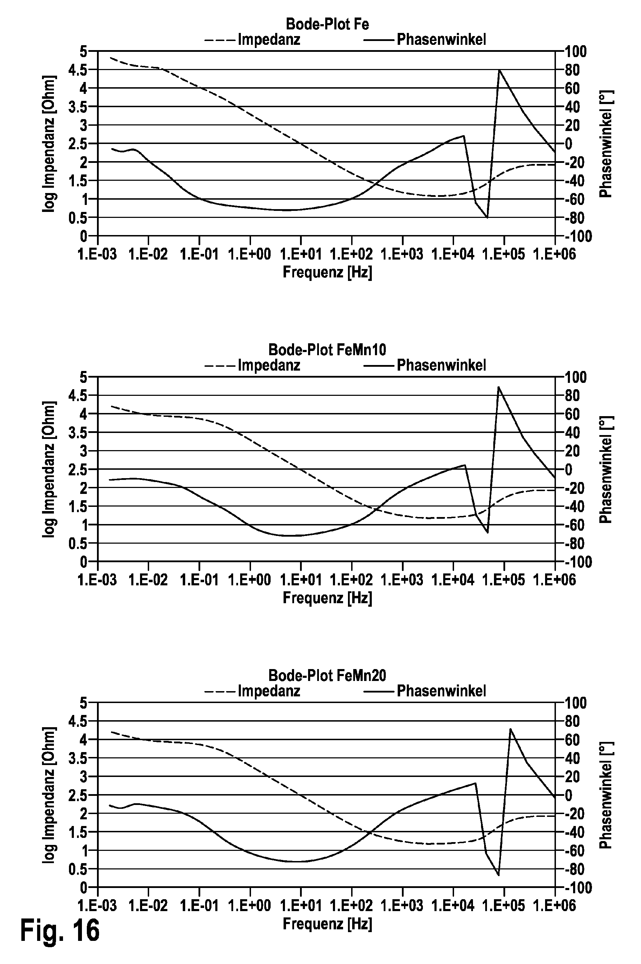

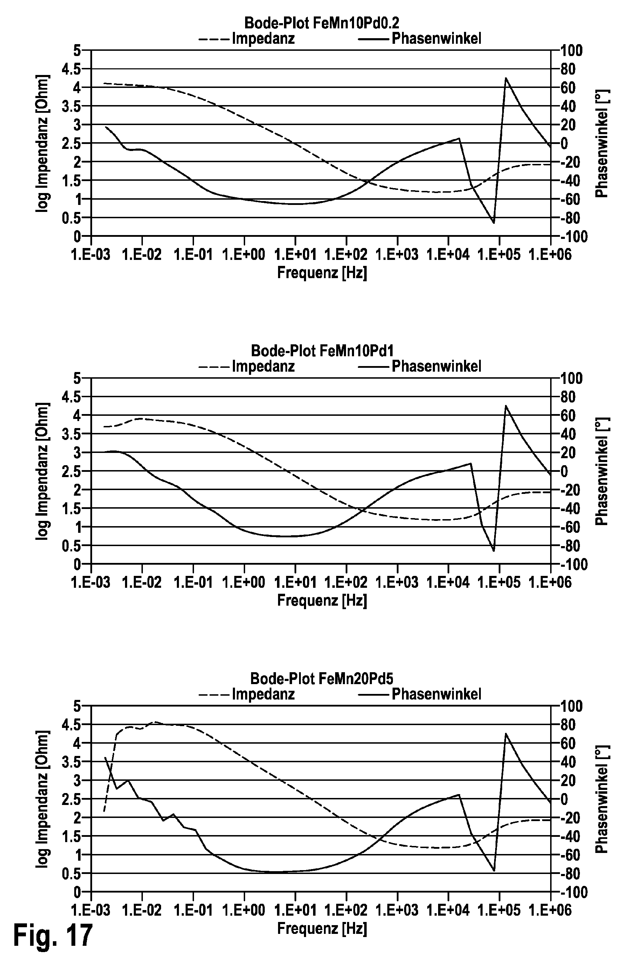

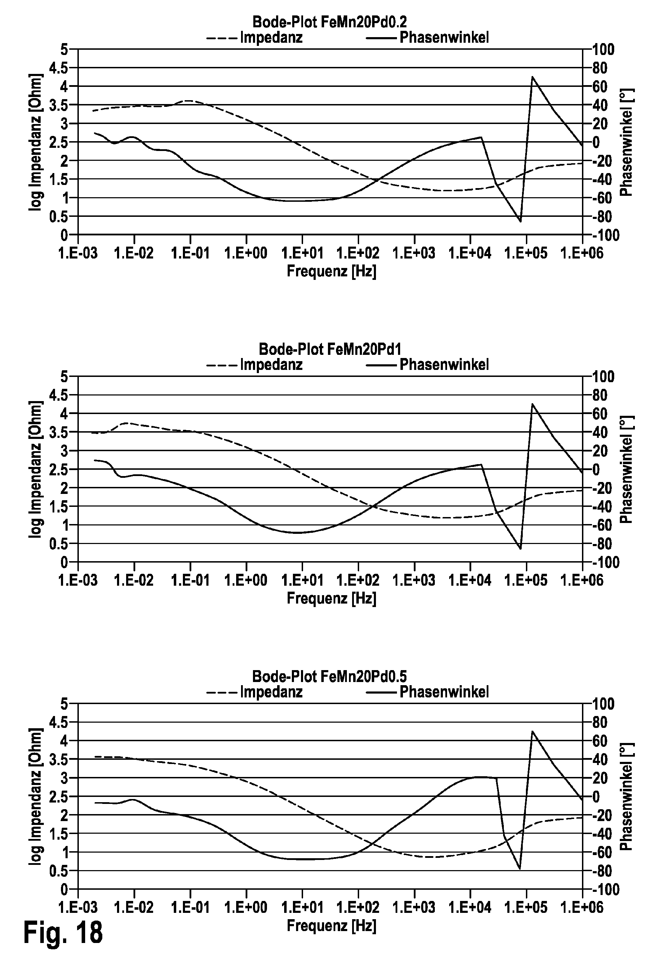

Fig. 16 zeigt Bode-Plots der untersuchten Pd-freien Legierungen (1100°C/2h + 700°C/1h). Fig. 17 zeigt Bode-Plots der untersuchten Legierungen mit 10% Mn (1100°C/2h + 700°C/1h). Fig. 18 zeigt Bode-Plots der untersuchten Legierungen mit 20% Mn (1100°C/2h + 700°C/1h).

Die EIS Ergebnisse zeigen, dass durch das Zulegieren von Mangan und Pd die Korrosionsempfindlichkeit offensichtlich signifikant zunimmt. Bei 20% Mangan und 5% Palladium beispielsweise zeigt sich der tiefste Wert, der mehr als den Faktor 10 kleiner ist als der von Reineisen. Es kann damit von einer korrosionsfördernden Wirkung von Mn und Pd ausgegangen werden. Dies wird auch von den Degradationsversuchen in SBF bestätigt, die im nächsten Abschnitt beschrieben werden.

### Degradationsversuche in simulierter Körperflüssigkeit (SBF = Simulated Body Fluid)

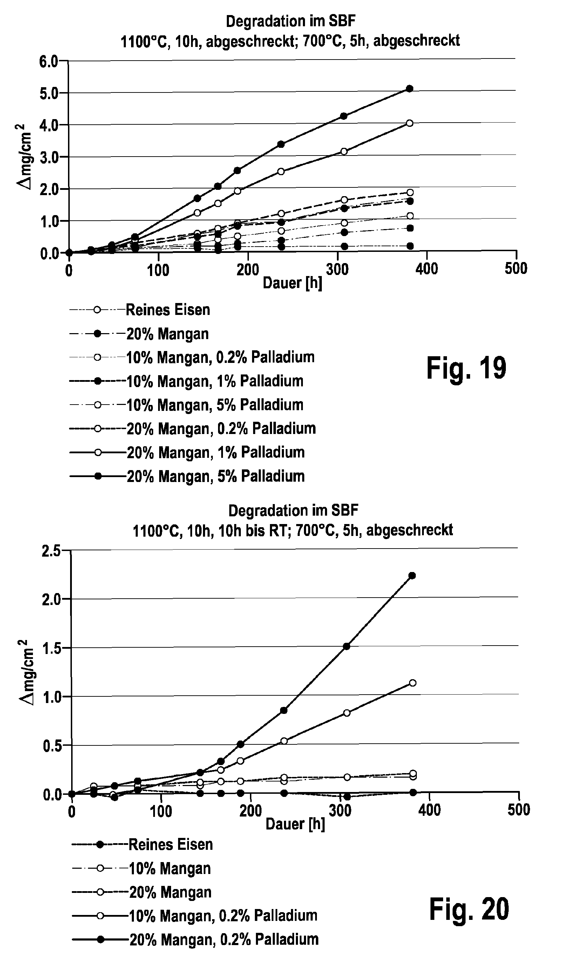

Fig. 19 zeigt den Masseverlust in SBF (1100°C/10h/H₂O + 700°C/5h). Fig. 20 zeigt den Masseverlust in SBF (1100°C/10h/langsam + 700°C/5h).

Die Ergebnisse der Auslagerungsversuche in SBF sind in den Abbildungen der Figuren 19 und 20 dargestellt. Es fällt auf, dass sämtliche manganhaltigen Proben schneller abgebaut werden als reines Eisen. Mit steigendem Pd-Gehalt nimmt die Korrosionsgeschwindigkeit zu. Die Art der Wärmebehandlung beeinflusst dabei stark die jeweiligen Abbauraten der Legierungen. Es ist anzunehmen, dass die Ergebnisse von der Elementsegregation mit bestimmt werden. Durch optische Begutachtung konnte gezeigt werden, dass die Spaltkorrosion dominiert.

Manganhaltige Eisenlegierungen zeigen somit insgesamt eine bedeutend tiefere Korrosionsresistenz als reines Eisen. Der Mangan- und Palladiumgehalt sowie die Art der Wärmebehandlung beeinflussen die Gefügeausbildung (martensitisch bzw. austenitisch), die physikalischen Eigenschaften (magnetisch/unmagnetisch bzw. Curietemperatur) und mechanischen Eigenschaften sowie die Korrosionsrate.

### Zugversuchsdaten zur Legierungsreihe FeMn(10-20)Pd1

Die Legierungen FeMn10Pd1, FeMn15Pd1 und FeMn20Pd1 wurden analog zu den oben beschriebenen Vorschriften hergestellt. Der Ausgangszustand der Legierungen war "lösungsgeglüht", d. h. es erfolgte eine langsame Ofenabkühlung (ca. 100°C/h) von 850°C.

Im Ausgangszustand ist die Legierung FeMn10Pd1 martensitisch (alpha-Martensit) und damit ferromagnetisch; die Legierung FeMn15Pd1 ist mehrphasig (alpha-Martensit, Austenit und epsilon Martensit) und damit leicht ferromagnetisch; die Legierung FeMn20Pd1 ist zweipahsig austenitisch + epsilon-Martensit und paramagnetisch (ganz leicht ferromagnetisch - epsilon Martensit ist zwar paramagnetisch, aber Mn hat eine spezielle, sehr komplexe Spinstruktur und ist bezüglich magnetische Eigenschaften schwer zu verstehen).

Es wurde nun überraschenderweise gefunden, dass beim Zugversuch eine dehnungsinduzierte Phasenumwandlung stattfindet und der ferromagnetische Anteil bei FeMn15Pd1 und FeMn20Pd1 deutlich zunimmt. Da somit alle Varianten im verformten Zustand ferromagnetisch sind, kann man diese Eigenschaft (Artefaktbildung bei bildgebenden Verfahren) zur Überwachung der Degradation nutzen.

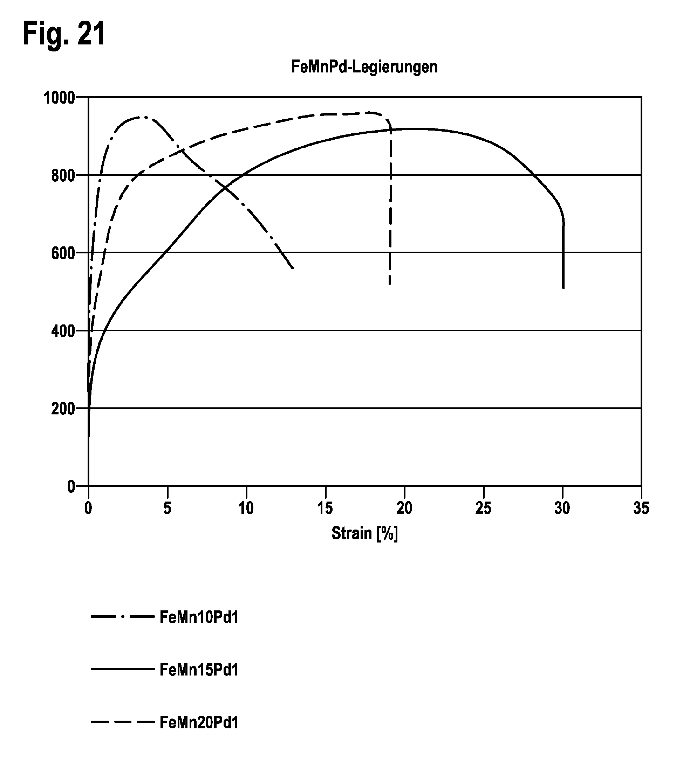

Die Spannungs-Dehnungs-Kurven zeigen deutlich das ausgeprägte Strain-hardening (Fig. 21). Bei FeMn15Pd1 und FeMn20Pd1 kommen wir bei 15% Dehnung (balooning) auf Werte von über 800 MPa, was Voraussetzung für ein sehr filigranes Design eines Stents ist (Reduktion der Strebendicke). Bei der Legierung FeMn15Pd1 ist eine Streckgrenze im Bereich von 350 MPa und eine maximale Festigkeit (Zugfestigkeit) im Bereich von 900 MPa festzustellen.

## Patentansprüche

1. Implantat mit einem Grundkörper bestehend ganz oder in Teilen
(i) aus einer biokorrodierbaren Eisenlegierung der Formel (1):
Fe-P (1)
wobei ein Anteil von P an der Legierung 0,01 bis 5 Gew.% beträgt und Fe sowie herstellungsbedingte Verunreinigungen den auf 100 Gew.% verbleibenden Restanteil an der Legierung einnehmen; oder
(ii) aus einer biokorrodierbaren Eisenlegierung der Formel (2):
Fe-Mn-X (2)
wobei ein Anteil von Mn an der Legierung 5 bis 30 Gew.% beträgt, X für ein oder mehrere Elemente ausgewählt aus der Gruppe Pt, Pd, Ir, Rh, Re, Ru und Os steht und ein Anteil von X an der Legierung 0,01 bis 10 Gew.% beträgt und Fe sowie herstellungsbedingte Verunreinigungen den auf 100 Gew.% verbleibenden Restanteil an der Legierung einnehmen; oder
(iii) aus einer biokorrodierbaren Eisenlegierung der Formel (3):
Fe-Z (3)
wobei Z für ein oder mehrere Elemente ausgewählt aus der Gruppe Pt, Ir und Os steht und ein Anteil von Z an der Legierung 5 bis 30 Gew.% beträgt und Fe sowie herstellungsbedingte Verunreinigungen den auf 100 Gew.% verbleibenden Restanteil an der Legierung einnehmen.

2. Implantat nach Anspruch 1, bei dem der Anteil von Mn an der Eisenlegierung der Formel (2) 10 bis 27 Gew.% beträgt.

3. Implantat nach einem der vorhergehenden Ansprüche, bei dem die Eisenlegierung der Formel (2) zusätzlich N oder C in einem Anteil von 0 bis 0,8 Gew.% an der Legierung enthält.

4. Implantat nach einem der vorhergehenden Ansprüche, bei dem das Implantat ein Stent, ein Clip oder ein Okkluder ist.

5. Implantat nach einem der Ansprüche 1 bis 3, bei dem das Implantat als Implantat zur Hyperthermiebehandlung ausgebildet ist.

6. Verwendung einer biokorrodierbaren Eisenlegierung
(i) der Formel (1):
Fe-P (1)
wobei ein Anteil von P an der Legierung 0,01 bis 5 Gew.% beträgt und Fe sowie herstellungsbedingte Verunreinigungen den auf 100 Gew.% verbleibenden Restanteil an der Legierung einnehmen; oder
(ii) der Formel (2):
Fe-Mn-X (2)
wobei ein Anteil von Mn an der Legierung 5 bis 30 Gew.% beträgt, X für ein oder mehrere Elemente ausgewählt aus der Gruppe Pt, Pd, Ir, Rh, Re, Ru und Os steht und ein Anteil von X an der Legierung 0,01 bis 10 Gew.% beträgt und Fe sowie herstellungsbedingte Verunreinigungen den auf 100 Gew.% verbleibenden Restanteil an der Legierung einnehmen; oder
(iii) aus einer biokorrodierbaren Eisenlegierung der Formel (3):
Fe-Z (3)
wobei Z für ein oder mehrere Elemente ausgewählt aus der Gruppe Pt, Ir und Os steht und ein Anteil von Z an der Legierung 5 bis 30 Gew.% beträgt und Fe sowie herstellungsbedingte Verunreinigungen den auf 100 Gew.% verbleibenden Restanteil an der Legierung einnehmen;
zur Herstellung eines Implantats, das für die Hyperthermiebehandlung ausgebildet ist.

7. Verwendung einer biokorrodierbaren Eisenlegierung
(i) der Formel (1):
Fe-P (1)
wobei ein Anteil von P an der Legierung 0,01 bis 5 Gew.% beträgt und Fe sowie herstellungsbedingte Verunreinigungen den auf 100 Gew.% verbleibenden Restanteil an der Legierung einnehmen; oder
(ii) der Formel (2):
Fe-Mn-X (2)
wobei ein Anteil von Mn an der Legierung 5 bis 30 Gew.% beträgt, X für ein oder mehrere Elemente ausgewählt aus der Gruppe Pt, Pd, Ir, Rh, Re, Ru und Os steht und ein Anteil von X an der Legierung 0,01 bis 10 Gew.% beträgt und Fe sowie herstellungsbedingte Verunreinigungen den auf 100 Gew.% verbleibenden Restanteil an der Legierung einnehmen; oder
(iii) aus einer biokorrodierbaren Eisenlegierung der Formel (3):
Fe-Z (3)
wobei Z für ein oder mehrere Elemente ausgewählt aus der Gruppe Pt, Ir und Os steht und ein Anteil von Z an der Legierung 5 bis 30 Gew.% beträgt und Fe sowie herstellungsbedingte Verunreinigungen den auf 100 Gew.% verbleibenden Restanteil an der Legierung einnehmen;
zur Herstellung eines Implantats, das zur temporären Fixierung von Gewebe ausgebildet ist.

## Claims

1. An implant having a main body consisting entirely or in parts:
(i) of a biocorrodible iron alloy of formula (1)
Fe-P (1),
wherein the amount of P in the alloy is from 0.01 to 5 % by weight, and Fe plus impurities due to the production process account for the remainder of the alloy up to 100 % by weight; or
(ii) of a biocorrodible iron alloy of formula (2)
Fe-Mn-X (2),
wherein the amount of Mn in the alloy is from 5 to 30 % by weight, X stands for one or more elements selected from the group Pt, Pd, Ir, Rh, Re, Ru and Os, and an amount of X in the alloy is from 0.01 to 10 % by weight, and Fe plus impurities due to the production process account for the remainder of the alloy up to 100 % by weight; or
(iii) of a biocorrodible iron alloy of formula (3)
Fe-Z (3),
wherein Z stands for one or more elements selected from the group Pt, Ir and Os, and an amount of Z in the alloy is 5 to 30 % by weight, and Fe plus impurities due to the production process account for the remainder of the alloy up to 100 % by weight.

2. The implant according to Claim 1, wherein the amount of Mn in the iron alloy of formula (2) is 10 to 27 % by weight.

3. The implant according to one of the preceding claims, wherein the iron alloy of formula (2) additionally contains N or C in an amount of 0 to 0.8 % by weight in the alloy.

4. The implant according to one of the preceding claims, wherein the implant is a stent, a clip or an occluder.

5. The implant according to one of Claims 1 to 3, wherein the implant is designed as an implant for treatment of hyperthermia.

6. Use of a biocorrodible iron alloy
(i) of formula (1):
Fe-P (1),
wherein the amount of P in the alloy is 0.01 to 5 % by weight, and Fe plus impurities due to the production process account for the remainder of the alloy up to 100 % by weight; or
(ii) of formula (2)
Fe-Mn-X (2),
wherein the amount of Mn in the alloy is 5 to 30 % by weight, X stands for one or more elements selected from the group Pt, Pd, Ir, Rh, Re, Ru and Os, and the amount of X in the alloy is 0.01 to 10 % by weight, and Fe plus impurities due to the production process account for the remainder of the alloy up to 100 % by weight; or
(iii) of a biocorrodible iron alloy of formula (3)
Fe-Z (3),
wherein Z stands for one or more elements selected from the group Pt, Ir and Os, and a proportion of Z in the alloy is from 5 to 30 % by weight, and Fe plus impurities due to the production process account for the remainder of the alloy up to 100 % by weight;
for production of an implant designed for treatment of hyperthermia.

7. Use of a biocorrodible iron alloy
(i) of formula (1):
Fe-P (1),
wherein the amount of P in the alloy is 0.01 to 5 % by weight, and Fe plus impurities due to the production process account for the remainder of the alloy up to 100 % by weight; or
(ii) of formula (2)
Fe-Mn-X (2),
wherein the amount of Mn in the alloy is 5 to 30 % by weight, X stands for one or more elements selected from the group Pt, Pd, Ir, Rh, Re, Ru and Os, and the amount of X in the alloy is 0.01 to 10 % by weight, and Fe plus impurities due to the production process account for the remainder of the alloy up to 100 % by weight; or
(iii) of a biocorrodible iron alloy of formula (3)
Fe-Z (3),
wherein Z stands for one or more elements selected from the group Pt, Ir and Os, and the amount of Z in the alloy is 5 to 30 % by weight, and Fe plus impurities due to the production process account for the remainder of the alloy up to 100 % by weight;
for production of an implant designed for temporary fixation of tissue.

## Revendications

1. Implant comportant un corps de base constitué totalement ou en partie
(i) d'un alliage de fer pouvant être corrodé biologiquement selon la formule (1) :
FE-P (1),
dans laquelle la fraction de P dans l'alliage s'élève de 0,01 à 5 % en poids et où le Fe ainsi que les impuretés liées à la fabrication représentent la partie restante de l'alliage jusqu'à 100 % en poids, ou
(ii) d'un alliage de fer pouvant être corrodé biologiquement selon la formule (2) :
Fe-Mn-X (2),
dans laquelle la fraction de Mn dans l'alliage s'élève de 5 et 30 % en poids, X représente un ou plusieurs éléments choisis dans le groupe constitué par Pt, Pd, Ir, Rh, Re, Ru et Os et la proportion de X dans l'alliage s'élève entre 0,01 et 10 % en poids, et où le fer ainsi que les impuretés liées à la fabrication représentent la partie restante de l'alliage jusqu'à 100 % en poids ; ou
(iii) d'un alliage de fer pouvant être corrodé biologiquement selon la formule (3)
Fe-Z (3),
dans laquelle Z représente un ou plusieurs éléments choisis dans le groupe constitué par Pt, Ir et Os et la fraction de Z dans l'alliage s'élève entre 5 et 30 % en poids, et où le Fe ainsi que les impuretés liées à la fabrication représentent la partie restante de l'alliage jusqu'à 100 % en poids.

2. Implant selon la revendication 1, dans lequel la fraction de Mn dans l'alliage de fer selon la formule (2) se monte entre 10 et 27 % en poids.

3. Implant selon l'une des revendications précédentes, dans lequel l'alliage de fer de la formule (2) contient supplémentairement N ou C dans une fraction de 0 à 8 % en poids dans l'alliage.

4. Implant selon l'une des revendications précédentes, dans lequel l'implant est une endoprothèse, une agrafe chirurgicale ou un occludeur.

5. Implant selon l'une des revendications 1 à 3, dans lequel l'implant est conçu comme un implant pour le traitement de l'hyperthermie.

6. Utilisation d'un alliage de fer pouvant être corrodé biologiquement
(i) selon la formule (1) :
Fe-P (1),
dans laquelle la fraction de P dans l'alliage s'élève de 0,01 à 5 % en poids et où le Fe ainsi que les impuretés liées à la fabrication représentent la partie restante de l'alliage jusqu'à 100 % en poids, ou
(ii) d'un alliage de fer pouvant être corrodé biologiquement selon la formule (2) :
Fe-Mn-X (2),
dans laquelle la fraction de Mn dans l'alliage s'élève entre 5 et 30 % en poids, X représente un ou plusieurs éléments choisis dans le groupe constitué par Pt, Pd, Ir, Rh, Re, Ru et Os et la fraction de X dans l'alliage s'élève entre 0,01 et 10 % en poids, et où le fer ainsi que les impuretés liées à la fabrication représentent la partie restante de l'alliage jusqu'à 100 % en poids ; ou
(iii) d'un alliage de fer pouvant être corrodé biologiquement selon la formule (3)
Fe-Z (3),
dans laquelle Z représente un ou plusieurs éléments choisis dans le groupe constitué de Pt, Ir et Os et la fraction de Z dans l'alliage s'élève entre 5 et 30 % en poids, et où le Fe ainsi que les impuretés liées à la fabrication représentent la partie restante de l'alliage jusqu'à 100 % en poids;
pour la fabrication d'un implant qui est conçu pour le traitement d'une hyperthermie.

7. Utilisation d'un alliage de fer pouvant être corrodé biologiquement
(i) selon la formule (1) :
Fe-P (1),
dans laquelle la fraction de P dans l'alliage s'élève entre 0,01 à 5 % en poids et où le Fe ainsi que les impuretés liées à la fabrication représentent la partie restante de l'alliage jusqu'à 100 % en poids, ou
(ii) d'un alliage de fer pouvant être corrodé biologiquement selon la formule (2) :
Fe-Mn-X (2),
dans laquelle la fraction de Mn dans l'alliage s'élève entre 5 et 30 % en poids, X représente un ou plusieurs éléments choisis dans le groupe constitué par Pt, Pd, Ir, Rh, Re, Ru et Os et la fraction de X dans l'alliage s'élève entre 0,01 et 10 % en poids, et où le fer ainsi que les impuretés liées à la fabrication représentent la partie restante de l'alliage jusqu'à 100 % en poids ; ou
(iii) d'un alliage de fer pouvant être corrodé biologiquement selon la formule (3)
Fe-Z (3),
dans laquelle Z représente un ou plusieurs éléments choisis dans le groupe constitué de Pt, Ir et Os et la fraction de Z dans l'alliage s'élève entre 5 et 30 % en poids, et où le Fe ainsi que les impuretés liées à la fabrication représentent la partie restante de l'alliage jusqu'à 100 % en poids ;
pour la fabrication d'un implant qui est conçu pour la fixation temporaire de tissus.
